# EUROPEAN PATENT APPLICATION

(11) **EP 4 434 351 A1**
(43) Date of publication of application: **25.09.2024**
(21) Application number: 22896106.6
(22) Date of filing: 17.11.2022
(51) Int. Cl.: A23K 50/50, A23K 20/147, A23K 20/163, A23K 20/158, A23K 20/142

(54) **FEED COMPOSITION FOR CONSTRUCTION OF STEATOHEPATITIS ANIMAL MODEL**

(30) Priority: 18.11.2021 KR 20210159324
(71) Applicant: Industry-Academic Cooperation Foundation, Yonsei University, Seoul 03722 (KR)
(72) Inventor: LEE, Yong Ho, Seoul 06518 (KR); KIM, Eun Ran, Incheon 21994 (KR)
(74) Representative: Müller, Christian Stefan Gerd
(86) International application number: PCT/KR2022/018231
(87) International publication number: WO 2023/090909

(57) **Abstract**

The present invention relates to a composition for inducing liver disease in a mammal and a mammal with a liver disease induced using the same. The animal model of the present invention has been found to be an excellent animal model in which fatty liver, hepatic fibrosis, and insulin resistance are evenly induced, and which successfully reproduces the progression of a series of severe liver diseases, from hepatitis and excessive fibrogenesis in liver tissue through tissue hardening (cirrhosis) to liver cancer, and reflects the characteristics of each stage with high reliability. Accordingly, the present invention may be useful as a means for developing a therapeutic agent for chronic liver disease as well as studying the molecular and circulatory mechanisms of each stage of liver disease.

## Description

### Technical Field

The present invention relates to a feed composition for establishing an animal model of liver disease, specifically a steatohepatitis-liver cancer continuum.

### Background Art

Chronic liver disease, which has a particularly high prevalence and mortality rate in Korea, is a serious condition that starts with non-alcoholic steatohepatitis, progresses to cirrhosis, and then leads to liver cancer through various complications that determine the prognosis of liver disease. Preventing progression from chronic hepatitis to cirrhosis is one of the ultimate goals for treating liver diseases caused by abnormal lipid metabolism. In addition, since cirrhosis occurs through the process of hepatic fibrosis that is sustained by various causes, effectively blocking and inhibiting the progression of hepatic fibrosis is also the most basic and important step in preventing serious liver diseases such as liver cancer.

Hepatic fibrosis is caused by excessive deposition of extracellular matrix (ECM) in liver tissue due to chronic intrahepatic inflammation, and if this excessive deposition of ECM persists, it eventually progresses to cirrhosis accompanied by structural deformation of the liver and a reduction in the number of hepatocytes. Although studies on therapeutic agents that effectively blocks this process from steatohepatitis through hepatic fibrosis to cirrhosis have been actively conducted, a therapeutic candidate substance that effectively alleviates fibrosis and has safety suitable for long-term administration, has not been developed. This is also due to the absence of an efficient animal model that can reproduce treatment responsiveness with high accuracy in clinical trials.

Throughout the present specification, a number of publications and patent documents are referred to and cited. The disclosure of these cited publications and patent documents is incorporated herein by reference in its entirety to more clearly describe the state of the art to which the present invention pertains and the content of the present invention.

### [Prior Art Documents]

(Patent Document 1) KR 10-2017-0028635 A

### DISCLOSURE

### Technical Problem

The present inventors have made extensive research efforts to develop an animal model that efficiently reproduces the progression of a series of pathological stages from inflammation to fibrosis and tissue sclerosis in liver tissue. As a result, the present inventors have found that, when a diet adjusted to have protein, carbohydrate, and fat contents accounting for 13 to 17%, 38 to 42%, and 43 to 47%, respectively, of the total calories of the diet is fed to a mammal, fatty liver, hepatic fibrosis, and insulin resistance are all efficiently induced. This allows the progression of a series of liver disease stages from nonalcoholic steatohepatitis in humans through cirrhosis and hepatic fibrosis to liver cancer in humans is successfully reproduced in the mammal, thereby completing the present invention.

Therefore, an object of the present invention is to provide a feed composition for inducing liver disease in a mammal.

Another object of the present invention is to provide a method of producing a mammal with induced liver disease using the composition.

Still another object of the present invention is to provide a mammal with induced liver disease, produced using the method.

Other objects and advantages of the present invention will be more apparent from the following detailed description, the appended claims, and the accompanying drawings.

### Technical Solution

According to one aspect of the present invention, the present invention provides a feed composition for inducing in a mammal a liver disease selected from the group consisting of steatohepatitis, hepatic fibrosis, cirrhosis and liver cancer, the feed composition comprising proteins, carbohydrates, and fats accounting for 13 to 17%, 38 to 42%, and 43 to 47%, respectively, of the total calories in the composition.

The present inventors have made extensive research efforts to develop an animal model that efficiently reproduces the progression of a series of pathological stages from inflammation to fibrosis and tissue sclerosis in liver tissue. As a result, the present inventors have found that, when a diet adjusted to have protein, carbohydrate and fat contents accounting for 13 to 17%, 38 to 42%, and 43 to 47%, respectively, of the total calories of the diet is fed to a mammal, fatty liver, hepatic fibrosis, and insulin resistance are all efficiently induced, so that the progression of a series of liver disease stages from non-alcoholic steatohepatitis in humans through cirrhosis and hepatic fibrosis to liver cancer in humans is successfully reproduced in the mammal.

In the present specification, the term "mammal" refers to all animals belonging to the mammalian class, excluding humans. Examples of the mammals include, but are not limited to, mice, rats, guinea pigs, dogs, cats, horses, cows, pigs, monkeys, chimpanzees, baboons, or rhesus monkeys. Specifically, the mammals are rodents.

In the present specification, the term "animal model" refers to a non-human animal that has been directly or indirectly artificially modified to have symptoms or phenotypes of liver disease. Specifically, the term "animal model" refers to an animal that has symptoms of acquired or non-genetic metabolic liver disease induced by oral intake of the dietary composition of the present invention and reflects the overall biological process ranging from non-alcoholic steatohepatitis through cirrhosis and hepatic fibrosis to liver cancer.

In the present specification, the term "feed" refers to any natural or artificial diet, meal or components of such diets or meals, intended or suitable for being eaten, taken in, and digested, by an animal to be raised. The composition of the present invention may be used by itself as a meal or diet for the target mammal, or it may be used as a feed additive that is added to other feed ingredients. The "feed composition" of the present invention refers to a composition whose functional ingredients or compositions have been artificially adjusted for the purpose of expressing a desired phenotype or reproducing a human disease, rather than improving the production of animals to be raised.

In the present specification, the term "steatohepatitis" refers to chronic inflammation in liver tissue caused by intrahepatic fat accumulation. Steatohepatitis is a pathological condition that is distinct from hepatic steatosis, in which an abnormal level of fat is simply maintained in liver tissue or hepatocytes for a long time. Symptoms of steatohepatitis cannot be alleviated or eliminated by simply reducing lipids, and the fundamental treatment of steatohepatitis is possible only by the control of activated inflammatory cytokines and reactive oxygen species.

In the present specification, the term "hepatic fibrosis" refers to a disease in which excessive fibrous connective tissue is formed in liver tissue by excessive deposition of the extracellular matrix due to chronic inflammation in the liver, resulting in damage to the normal structure and function of the liver.

In the present specification, the term "cirrhosis" refers to a pathological condition in which normal liver tissue is lost as inflammation and extracellular matrix deposition in the liver tissue continue for a long time, and liver tissue does not function normally as scar tissue replaces the lost liver tissue

Hepatic fibrosis and cirrhosis are not independent diseases with separate etiologies, but are continuous diseases progressing from chronic hepatitis through fibrosis to cirrhosis, ultimately leading to liver cancer. To date, various therapeutic agents for metabolic diseases that have been commercially available because of their demonstrated lipid-lowering effect have failed to effectively alleviate hepatic fibrosis and hardening of liver tissue.

According to a specific embodiment of the present invention, the composition of the present invention comprises proteins, carbohydrates, and fats accounting for 14 to 16%, 39 to 41%, and 44 to 46%, respectively, of the total calories in the composition. More specifically, the composition of the present invention comprises proteins, carbohydrates, and fats accounting for about 15%, about 40%, and about 45%, respectively, of the total calories.

According to a specific embodiment of the present invention, the composition comprises, based on the total weight of the composition, 15 to 19 w/w% of casein and 0.2 to 0.3 w/w% of L-cysteine.

More specifically, the composition comprises 16 to 18 w/w % of casein and 0.24 to 0.28 w/w % of L-cysteine. Most specifically, the composition comprises 17.2 to 17.7 w/w % of casein and 0.25 to 0.27 w/w % of L-cysteine.

According to a specific embodiment of the present invention, the composition further comprises, based on the total weight of the composition, 21 to 25 w/w % of fructose and 10 to 12 w/w % of sucrose. More specifically, the composition further comprises 22 to 24 w/w % of fructose and 10.5 to 11.5 w/w % of sucrose. Most specifically, the composition further comprises about 23 w/w % of fructose and about 11 w/w % of cysteine.

According to a specific embodiment of the present invention, the composition further comprises, based on the total weight of the composition, 16 to 20 w/w% of lard.

More specifically, the composition further comprises 17 to 19 w/w% of lard. Most specifically, the composition further comprises about 18 w/w% of lard.

According to a specific embodiment of the present invention, the composition further comprises, based on the total weight of the composition, 0.5 to 0.7 w/w% of cholesterol.

According to a specific embodiment of the present invention, the composition does not comprise choline. Choline is a water-soluble vitamin that exists in the form of phospholipid phosphatidylcholine and is a raw material for lecithin, which makes up cell membranes in the body, and acetylcholine, which lowers blood pressure. In the present specification, "does not comprise choline" is meant to include not only a case where choline is not present at a detectable level in the composition of the present invention, but also a case where choline is present in an amount less than a nutritionally effective amount.

According to a specific embodiment of the present invention, the composition of the present invention increases the expression of at least one gene, selected from the group consisting of α-SMA, COL1A1, TNF-α, MCP-1, p21, p16, CXCL1, MMP13 and ICAM1, in liver tissue.

As shown in the Examples below, it was found that the dietary composition of the present invention increased the expression of the major inflammatory factors TNF-α and MCP-1, the fibrosis factors α-SMA and COL1A1, and the senescence factors p21, p16, CXCL1, MMP13, and ICAM1, thereby efficiently inducing the progression of inflammation and cell senescence and the resulting fibrosis, thus reproducing with high reliability the symptoms of human nonalcoholic steatohepatitis in which fat accumulation, fibrosis and insulin resistance all worsen, without exhibiting the simple effect of increasing only lipid accumulation.

In the present specification, the phrase "increase the expression" means that the expression level of a protein or gene that is a marker or cause of inflammation, fibrosis, or senescence increases so that pathological inflammation, fibrosis and senescence in liver tissue progress, accelerate, or worsen or the risk thereof increases. Specifically, the phrase may mean that the expression level increases by at least 20%, more specifically at least 30%, even more specifically at least 40%, compared to that in a control group.

According to another aspect of the present invention, the present invention provides a method for producing an animal with an induced liver disease selected from the group consisting of steatohepatitis, hepatic fibrosis, cirrhosis, and liver cancer, the method comprising a step of administering the above-described feed composition of the present invention to a mammal.

In the present specification, the term "administration" or "administering" means administering a nutritionally effective amount of the composition of the present invention directly to a subject so that the same amount is formed in the subject's body. Administration in the present invention is specifically oral administration, and thus the term "administering" has the same meaning as "feeding".

In the present invention, the term "nutritionally effective amount" refers to an amount of the feed composition of the present invention that contains active ingredients sufficient to express a desired phenotype in a subject.

The active ingredients in the composition of the present invention, for example, casein, L-cysteine, fructose, sucrose, and lard, are all contained in the same feed formulation (unitary diet) or are contained in different feed formulations, and the method of the present invention may be performed by feeding these active ingredients simultaneously or sequentially.

According to a specific embodiment of the present invention, the method is performed by feeding the mammal the feed composition of the present invention in an amount of 2.5 to 4 g/kg every day. More specifically, the method is performed by feeding the feed composition in an amount of 2.5 to 3.5 g/kg every day, most specifically about 3 g/kg every day.

According to a specific embodiment of the present invention, the method is performed by feeding the mammal the feed composition of the present invention for 30 to 400 days.

More specifically, the feed composition may be fed for 30 to 40 days to induce steatohepatitis in the mammal.

More specifically, the composition may be fed for 80 to 150 days, most specifically 90 to 140 days, to induce hepatic fibrosis in the mammal.

More specifically, the composition may be fed for 300 to 400 days, even more specifically 340 to 380 days, most specifically 350 to 370 days, to induce liver cancer in the mammal.

According to still another aspect of the present invention, the present invention provides a mammal with an induced liver disease selected from the group consisting of steatohepatitis, hepatic fibrosis, cirrhosis, and liver cancer, produced by the above-described method of the present invention.

Since the feed composition of the present invention, the liver disease that may be induced using the same, and the target mammal have already been described in detail, the description thereof will be omitted to avoid excessive overlapping.

According to a specific embodiment of the present invention, the mammal of the present invention exhibits continuous disease progression from steatohepatitis to liver cancer.

The animal model of the present invention is not only an animal model in which fatty liver, inflammation, liver fibrosis, insulin resistance, and cell senescence are evenly induced, but also shows a high rate of liver cancer development over the course of the feeding period, and thus it successfully reproduces the step-by-step progression of human liver disease, starting from hepatitis and leading to hepatic fibrosis, cirrhosis, and liver cancer. The conventionally used AMLN (amylin liver NASH) diet induces fatty liver and hepatic fibrosis to some extent, but does not induce insulin resistance at all, and the CD-HFD (choline-deficient high-fat diet) induces fatty liver and insulin resistance, but has the disadvantage of not inducing the progression of hepatic fibrosis. For this reason, in order to reproduce the continuous progression of liver disease as in humans, it was necessary to feed different diets at different stages or to provide separate stimulation over time. However, the animal model of the present invention is an animal model in which all stages of liver disease are induced over time by feeding only the above-described feed composition of the present invention, and thus the animal model may be applied as an efficient means for studying pathological mechanisms, screening therapeutic agents, and evaluating drugs *in vivo.*

### Advantageous Effects

The features and advantages of the present invention are summarized as follows:
(a) The present invention provides a composition for inducing liver disease in a mammal and a mammal with a liver disease induced using the same.
(b) The animal model of the present invention has been found to be an excellent animal model in which fatty liver, hepatic fibrosis, insulin resistance, and cell senescence are evenly induced, and which successfully reproduces the progression of a series of severe liver diseases, from hepatitis and excessive fibrogenesis in liver tissue through tissue hardening (cirrhosis) to liver cancer, and reflects the characteristics of each stage with high reliability.
(c) The present invention may be useful as a means for developing a therapeutic agent for chronic liver disease as well as studying the molecular and circulatory mechanisms of each stage of liver disease.

### Brief Description of Drawings

FIG. 1 shows changes in mouse body weight following feeding of each of a normal diet (chow), a choline-deficient high-fat diet (CD-HFD), and a dietary composition (New diet) of the present invention.
FIG. 2 shows changes in mouse liver weight (FIGS. 2a and 2b) and changes in blood ALT (alanine aminotransferase) level (FIGS. 2c and 2d) following feeding of each of the normal diet, the choline-deficient high-fat diet, and the dietary composition of the present invention.
FIG. 3 shows liver morphology changes and progression of fibrosis following feeding of each of the normal diet and the dietary composition of the present invention.
FIG. 4 shows histological lesion features of NASH such as inflammation (FIG. 4a) and changes in expression of αSMA, a hepatic fibrosis-related protein (FIG. 4b), following feeding of each of the normal diet and the dietary composition of the present invention.
FIG. 5 shows the results of an intraperitoneal glucose tolerance test (IPGTT) performed to measure changes in insulin resistance following feeding of each of the normal diet and the dietary composition of the present invention.
FIG. 6 shows changes in intrahepatic triglyceride accumulation following feeding of each of the normal diet and the dietary composition of the present invention.
FIG. 7 is a heat map showing changes in gene expression following feeding of each of the normal diet and the dietary composition of the present invention.
FIG. 8 shows changes in mRNA expression levels of TNFα (FIG. 8a), MCP1 (FIG. 8b), Col1a1 (FIG. 8c), αSMA (FIG. 8d), p21 (FIG. 8e), p16 (FIG. 8f), CXCL1 (FIG. 8g), MMP3 (FIG. 8h), and ICAM1 (FIG. 8i) following feeding of each of the normal diet and the dietary composition of the present invention.
FIG. 9 shows changes in the expression of the senescence-related factor SA-β-gal in liver tissue following feeding of each of the normal diet and the dietary composition of the present invention.
FIGS. 10a to 10c show T cell profiling following feeding of each of the normal diet and the dietary composition of the present invention.
FIGS. 11a and 11b show macrophage profiling following feeding of each of the normal diet and the dietary composition of the present invention.
FIG. 12 shows changes in mouse body weight over 50 weeks following feeding of each of the normal diet and the dietary composition of the present invention.
FIG. 13 shows the formation of liver cancer following feeding of each of the choline-deficient high-fat diet (CD-HFD) and the dietary composition of the present invention, and shows the appearance of the formed tumor (FIG. 13a) and the number of tumors and the tumor size (FIG. 13b).

### Best Mode

One embodiment of the present invention is directed to a feed composition for inducing in a mammal a liver disease selected from the group consisting of steatohepatitis, hepatic fibrosis, cirrhosis and liver cancer, the feed composition comprising proteins, carbohydrates, and fats accounting for 13 to 17%, 38 to 42%, and 43 to 47%, respectively, of the total calories in the composition.

Another embodiment of the present invention is directed to a method for producing an animal with an induced liver disease selected from the group consisting of steatohepatitis, hepatic fibrosis, cirrhosis, and liver cancer, the method comprising a step of administering to a mammal the feed composition comprising proteins, carbohydrates, and fats accounting for 13 to 17%, 38 to 42%, and 43 to 47%, respectively, of the total calories in the composition.

Still another embodiment of the present invention is directed to a mammal with an induced liver disease selected from the group consisting of steatohepatitis, hepatic fibrosis, cirrhosis, and liver cancer, produced by the above-described method for producing an animal with an induced liver disease.

### Mode for Invention

Hereinafter, the present invention will be described in more detail by way of examples. These examples are only for explaining the present invention in more detail, and it will be apparent to those skilled in the art that the scope of the present invention according to the subject matter of the present invention is not limited by these examples.

### Examples

Table 1 below summarizes the contents of the main components in a diet (New diet) of the present invention, AMLN (amylin liver NASH) diet, and CD-HFD (choline-deficient high-fat diet).

**[Table 1]**

| | New diet | | AMLN | | CD-HFD | |
|---|---|---|---|---|---|---|
| | gm% | kcal% | gm% | kcal% | gm% | kcal% |
| Proteins | 18 | *15* | 22 | *20* | 23.6 | *20* |
| Carbohydrates | 47 | *40* | 45 | *40* | 41.3 | *35.1* |
| Fats | 24 | *45* | 20 | *40* | 23.5 | *44.9* |
| Total | | 100 | | 100 | | 100 |
| kcal/gm | 4.7 | | 4.5 | | 4.71 | |
| Components | gm | *kcal* | gm | *kcal* | gm | *kcal* |
| Casein | 150 | *600* | 200 | *800* | 200 | *800* |
| L-Cysteine | 2.3 | *9.2* | 3 | *12* | 3 | *12* |
| Corn starch | 0 | *0* | 0 | *0* | 72.8 | *291.2* |
| Maltodextrin 10 | 100 | *400* | 100 | *400* | 100 | *400* |
| Fructose | 200 | *800* | 200 | *800* | 0 | *0* |
| Sucrose | 96 | *384* | 96 | *384* | 172.8 | *691.2* |
| Cellulose, BW200 | 50 | *0* | 50 | *0* | 50 | *0* |
| Soybean oil | 25 | *225* | 25 | *225* | 25 | *225* |
| Lard | 155 | *1395* | 177.5 | *1598* | 177.5 | *1597.5* |
| Mineral Mix S10026 | 10 | *0* | 10 | *0* | 10 | *0* |
| Dicalcium phosphate | 13 | *0* | 13 | *0* | 13 | *0* |
| Calcium carbonate | 5.5 | *0* | 5.5 | *0* | 5.5 | *0* |
| Potassium citrate, 1H2O | 16.5 | *0* | 16.5 | *0* | 16.5 | *0* |
| Vitamin Mix V10001 | 10 | *40* | 10 | *40* | 10 | *40* |
| Choline bitartrate | 0 | *0* | 2 | *0* | 0 | *0* |
| Cholesterol | 4.3 | *0* | 18 | *0* | 4.3 | *0* |
| FD&C Yellow Dye#5 | 0.05 | *0* | 0.025 | *0* | 0.05 | *0* |
| FD&C Red Dye#40 | 0 | *0* | 0 | *0* | 0 | *0* |
| FD&C Blue Dye#1 | 0 | *0* | 0.025 | *0* | 0 | *0* |
| Total | 860.15 | *4056* | *904.05* | *4056* | 856.15 | *4057* |
| Additional cholesterol (gm%) | 0.5 | 0 | 2.0 | 0 | 0 | 0 |

### Body Weight Change According to Type of Diet

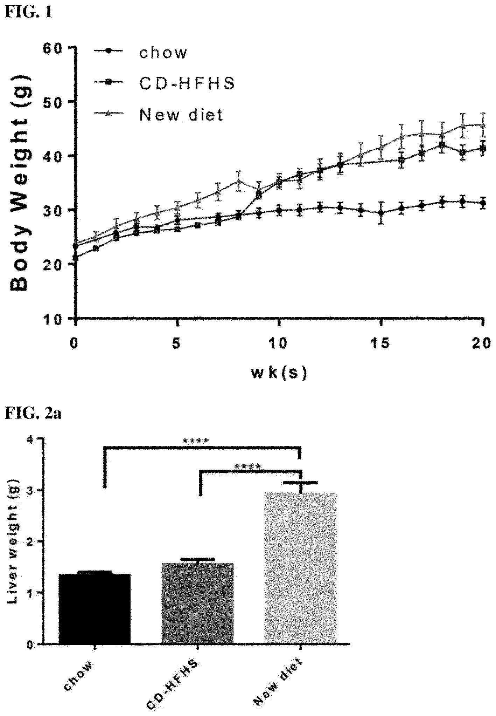

8-week-old C57BL6/J mice were fed a normal diet (chow, N=5), CD-HFHS (CDHFD (choline-deficient high-fat diet) + high fructose (128 mM) and high sucrose (55 mM) + water)) or a diet of the present invention, and the body weight of each mouse was measured at the same time every week. It was confirmed that the body weight gain significantly increased in the groups fed each of the NASH-inducing diet CD-HFHS and the diet (New diet) of the present invention compared to the mice fed chow (FIG. 1). At the end of the diet feeding (20 weeks), obesity, one of the pathological characteristics of NASH, was induced by each of 31.26 g of chow, 40.6 g of CH-HFHS, and 45.32 g of the diet of the present invention. In particular, the group fed the diet of the present invention was observed to exhibit a significant body weight gain effect compared to the previously known CD-HFHS (p<0.05 for main effect by diet, two-way analysis of variance).

### Changes in Liver Function According to Type of Diet

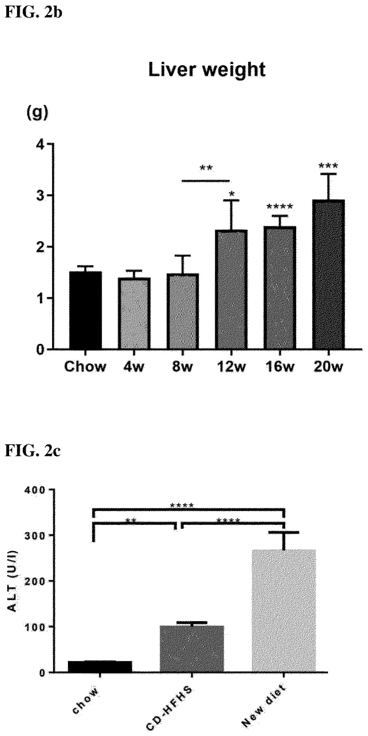

After feeding each of chow, CD-HFHS, and the diet of the present invention for 20 weeks, blood was removed by intracardiac bleeding, and then the total liver weight was measured. As a result, it was confirmed that the liver weight of the group fed the diet of the present invention significantly increased compared to that of the mice fed each of chow and CD-HFHS (FIG. 2a, ****p < 0.0001, Tukey's multiple comparative test). In addition, the total liver weight of the group fed each of chow and the diet of the present invention was measured at 4-week intervals, and as a result, it was confirmed that the liver weight of the group fed the diet of the present invention significantly increased from week 12 (FIG. 2b, *p<0.05, **p<0.01, ***p<0.005, ****p<0.0001, Student's t-test).

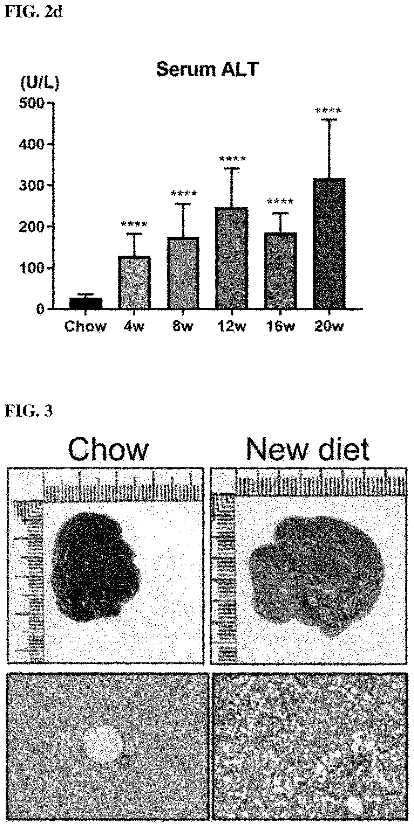

In addition, the alanine aminotransferase (ALT) level in the blood was measured by the ALT kit (FUJIFILM, GPT/ALT-PIII, 3250) and FDC4000i system using 10 µ serum isolated from the blood by centrifugation (3,000 rpm, 15°C, 15 min). As a result, it was observed that the ALT level in the blood, which is one of the markers of liver injury, significantly increased in the CD-HFHS group and the group fed the diet of the present invention compared to the chow-fed group. In particular, it was confirmed that the ALT level in the group fed the diet of the present invention was more than two times higher than that of the CD-HFHS group, and significantly increased starting from week 4 compared to the chow-fed group (FIGS. 2c and 2d, **p <0.01, ****p<0.0001, Tukey's multiple comparison test or Student's t-test).

### Progression of Hepatic Fibrosis According to Type of Diet

After 8-week-old C57BL6/J mice were fed each of chow, CD-HFHS, and the diet of the present invention for 20 weeks, the mice were sacrificed, the liver tissue was harvested from each mouse, fixed with 10% formaldehyde at 4°C for 24 hours, and then prepared into a paraffin block and a slide (10 µm). The prepared slide was deparaffinized and hydrated by immersion in xylene, xylene + ethanol (100%) solution, and ethanol (100*%*) for 10 minutes each. After dehydration, the tissue was sufficiently covered with Sirius Red/Fast Green staining solution (Chondrex, #90461) and stained for 30 minutes at room temperature. After 30 minutes, the slide was washed thoroughly with distilled water and dried, and then the tissue was sufficiently covered with Picrosirius Red F3BA Solution B (Polysciences, #24901B) and stained for 30 minutes at room temperature. After 30 minutes, the slide was washed thoroughly with distilled water, and staining was completed by covering the slide with a cover glass using VectaMount Permanent Mounting Medium (VECTOR, H-5000). After drying, the slide was observed using an optical microscope (Olympus, BX53) at 40x magnification and 100x magnification. As a result of observation, it was shown that, compared to the liver of the mouse fed chow, the liver of the mouse fed the diet of the present invention became hypertrophic and the red color became lighter due to fat accumulation (top of FIG. 3). In addition, as a result of evaluating the amount of collagen accumulation, which is a representative marker for the degree of fibrosis progression, formation of bridging fibrosis characteristic of human steatohepatitis patients was observed in the perivascular area in the mouse fed the diet of the present invention (FIG. 3).

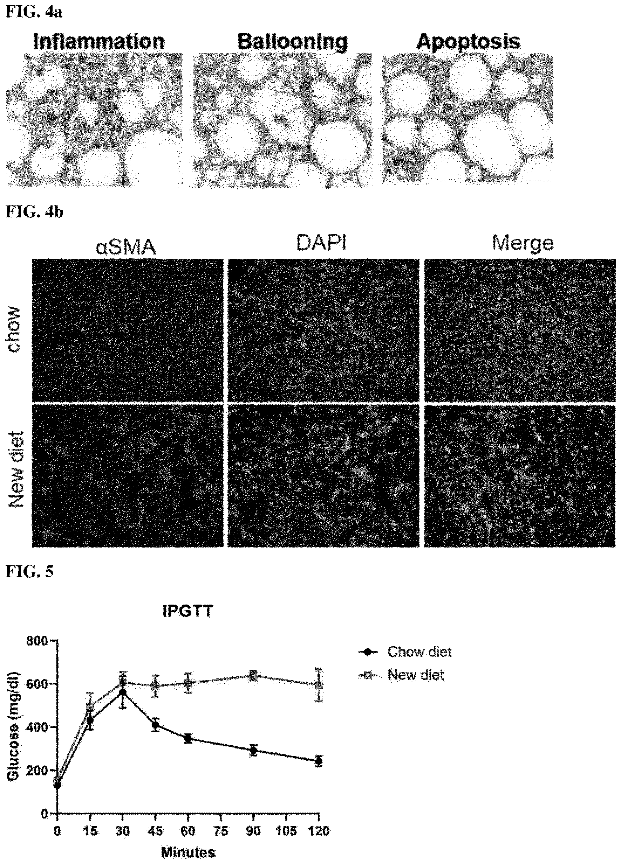

In addition, as a result of eosin & hematoxylin staining, it was confirmed that inflammation, ballooning, and apoptosis, which are the characteristics observed in human steatohepatitis patients, were well developed in the group fed the diet of the present invention (FIG. 4a).

In addition, in order to measure changes in the expression of the hepatic fibrosis-related protein αSMA (α-smooth muscle actin), the present inventors deparaffinized and hydrated the paraffin slide (4 µm) with xylene and ethanol, and then performed antigen retrieval by heating the slide in 1x sodium citrate buffer for 20 minutes. The slide was covered with 2.5% normal horse serum (VECTOR, VECTASTAIN KIT, 94010) and blocked by incubation at room temperature for 20 minutes, followed by avidin and biotin blocking steps (VECTOR, Avidin/Biotin Blocking Kit, SP-2001) at room temperature for 15 minutes each. The slide was incubated with anti-αSMA primary antibody (1:100, Abcam, Anti-alpha smooth muscle Actin antibody, ab5694) at room temperature for 1 hour, followed by incubation at 4°C overnight. Then, the slide was incubated with secondary antibody (1:200, Invitrogen, Alexa Fluor 594 donkey anti-rabbit IgG, A21207) at room temperature for 1 hour and 30 minutes under a light-shielded condition. To observe the nucleus, the slide was stained with antifade mounting medium with DAPI (VECTOR, H-1200), and staining was completed by covering the slide with a cover glass. After drying, the slide was observed using a fluorescence microscope (ZEISS, Axio Imager M2).

As a result of observation, it was confirmed that the expression of αSMA protein, a representative marker of hepatic fibrosis, significantly increased in the mouse fed the diet of the present invention compared to the mouse fed chow (FIG. 4b).

### Glucose Tolerance Test after Diet Feeding

8-week-old C57BL6/J mice fed chow or the diet of the present invention for 19 weeks were fasted for 6 hours before the experiment and injected intraperitoneally with glucose (2 g/kg, ROTH, D-(+)-glucose anhydrous, X997.2). The tip of the tail was cut off and blood glucose was measured at 0, 15, 30, 45, 60, 90, and 120 minutes. As a result of the glucose tolerance test, it was confirmed that the elevated blood glucose level in the mice fed the diet of the present invention was maintained up to 120 minutes, unlike the blood glucose level in the chow diet-fed mice, which decreased to a level close to the previous level at 120 minutes, suggesting that pancreatic beta-cell dysfunction was induced in the mice fed the diet of the present invention (FIG. 5).

### Observation of Intrahepatic Triglyceride Accumulation

200 µL of 5% Triton X-100 (SIGMA, TRITON X-100, X-100) was added to 30 mg of the liver tissue which was then homogenized. After heating at 100°C for 5 minutes and then cooling to room temperature was repeated twice, the supernatant collected by centrifugation (13,000 rpm, 4°C, 15 minutes) was used for analysis. Analysis was performed using the TG assay kit (Bioassay system, EnzyChrom^{™} Triglyceride Assay Kit, ETGA-200) according to the kit protocol.

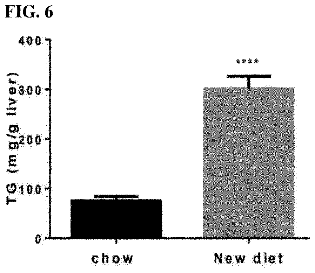

As a result of the analysis, it was confirmed that the level of intrahepatic triglyceride accumulation in the mice fed the diet of the present invention was more than three times higher than that in the mice fed the chow (FIG. 6).

### Changes in Gene Expression in Liver Tissue

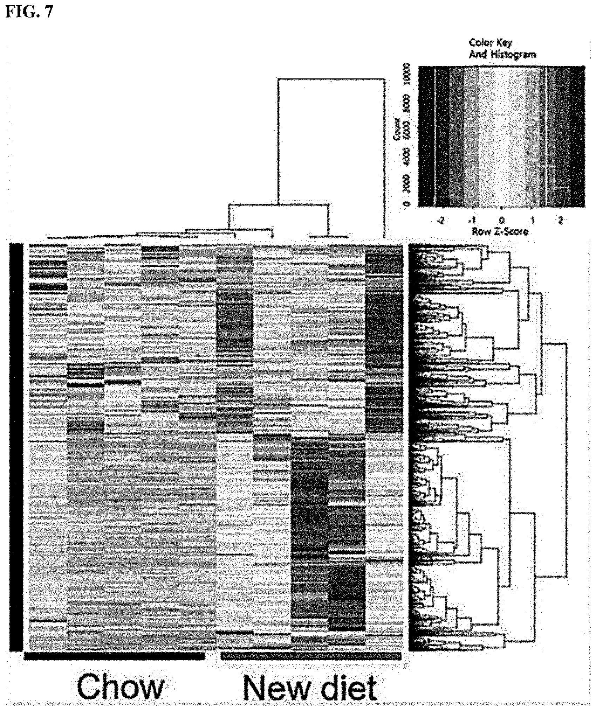

The 8-week-old C57BL6/J mice fed each of chow, CD-HFHS, and the diet of the present invention for 20 weeks were sacrificed, and the liver tissue was harvested from each mouse, placed in 1 ml of Trizol (Ambion, Trizol Reagent, 15596018), frozen in liquid nitrogen, and stored in an ultra-low temperature freezer at -80°C. After lysis, total RNA integrity of each sample was analyzed using the Agilent 2100 BioAnalyzer. If the RIN (RNA Integrity Number) value was greater than 6, it was considered to meet the library construction standards. After constructing a library using the TruSeq DNA kit (150 pair end), 12-Gb data was generated using the Illumina platform (NovaSeq), and analysis of differentially expressed genes (DEGs) was performed. As a result, it was observed that, in the group fed the diet of the present invention, the expression of 2,178 genes increased and the expression of 1,894 genes decreased (cutoff: fold change > 2 and p-value < 0.05) (FIG. 7).

### Changes in Gene Expression in Liver Tissue

The liver tissue was homogenized in 0.5 ml of Trizol (Ambion, Trizol Reagent, 15596018), and then 0.2 ml of chloroform (SIGMA-ALDRICH, Chloroform, C2432) was added thereto, followed by vortexing and then centrifugation (13,000 rpm, 4°C, 15 minutes). 0.2 ml of isopropanol was added to the collected supernatant, followed by incubation at room temperature for 30 minutes. Then, RNA was isolated from the supernatant by centrifugation (13,000 rpm, 4°C, 15 minutes). cDNA was synthesized from RNA using a cDNA kit (Applied Biosystems, High Capacity cDNA Reverse Transcription Kit, 4368813) and then used for qPCR analysis. qPCR was performed with the following primers using the SYBR Green (Applied Biosystems, PowerSYBR Green PCR Master Mix, 4367659):

**[Table 2]**

| Gene | | Primer sequence | SEQ ID NO. |
|---|---|---|---|
| TNFα | Forward | GCTACGACGTGGGCTACAG | SEQ ID NO: 1 |
| | Reverse | CCCTCACACTCAGATCATCTTCT | SEQ ID NO: 2 |
| MCP1 | Forward | TTAAAAACCTGGATCGGAACCA A | SEQ ID NO: 3 |
| | Reverse | GCATTAGCTTCAGATTTACGGGT | SEQ ID NO: 4 |
| Col1a1 | Forward | TAAGGGTCCCCAATGGTGAGA | SEQ ID NO: 5 |
| | Reverse | GGGTCCCTCGACTCCTACAT | SEQ ID NO: 6 |
| αSMA | Forward | GTCCCAGACATCAGGGAGTAA | SEQ ID NO: 7 |
| | Reverse | TCGGATACTTCAGCGTCAGGA | SEQ ID NO: 8 |
| p21 | Forward | GCAGACCACAGCGTATCCA | SEQ ID NO: 9 |
| | Reverse | AACAGGTCGGACATCACCAG | SEQ ID NO: 10 |
| p16 | Forward | CCCAACGCCCCGAACT | SEQ ID NO: 11 |
| | Reverse | GCAGAAGAGCTGCTGCTACGTG A | SEQ ID NO: 12 |
| CXCL1 | Forward | ACCGAAGTCATAGCCACACTC | SEQ ID NO: 13 |
| | Reverse | CTCCGTTACTTGGGGACACC | SEQ ID NO: 14 |
| MMP13 | Forward | AAGGGGATAACAGCCACTACAA | SEQ ID NO: 15 |
| | Reverse | ACCAACATAAAAATTAAGCCAA AT | SEQ ID NO: 16 |
| ICAM1 | Forward | CTTCCAGCTACCATCCCAAA | SEQ ID NO: 17 |
| | Reverse | CTTCAGAGGCAGGAAACAGG | SEQ ID NO: 18 |

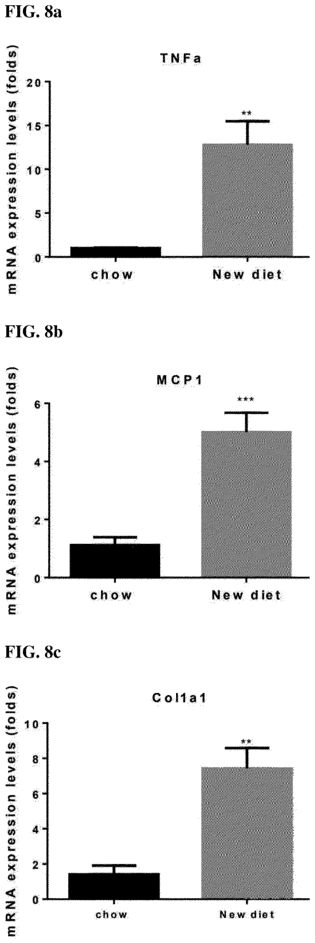

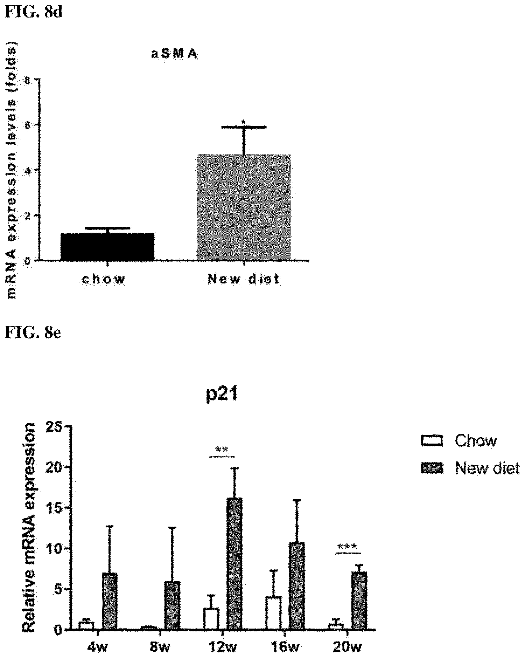

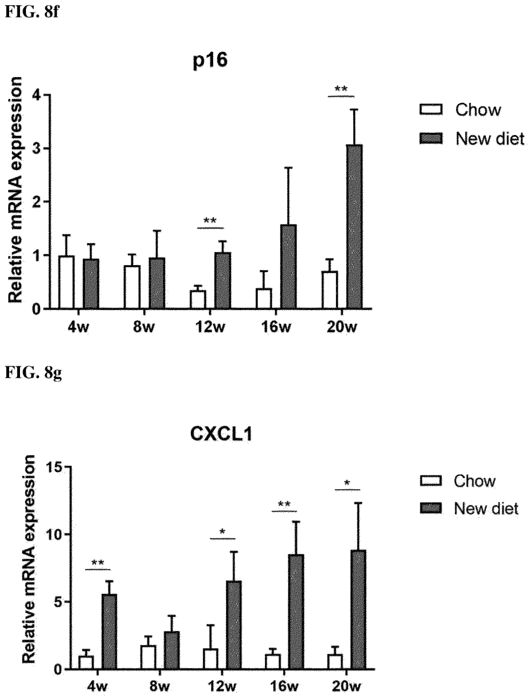

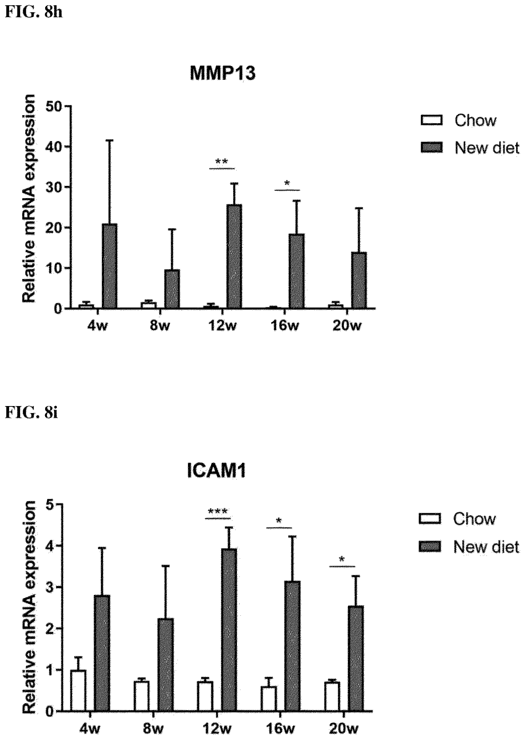

As a result of measurement, it was confirmed that the mRNA expression of the inflammation-related genes TNFα (α-tumor necrosis factor) and MCP1 (monocyte chemotactic protein-1) significantly increased in the mice fed the diet of the present invention compared to the mice fed chow (FIGS. 8a and 8b, ***p<0.001, **p<0.01, Student's t-test), and the mRNA expression of the liver fibrosis-related genes Col1a1 and αSMA (α-smooth muscle actin) was also significantly higher in the mice fed the diet of the present invention than in the mice fed chow (FIGS. 8c and 8d, **p<0.01, *p<0.05, Student's t-test). In addition, it was confirmed that the mRNA expression of the cell senescence-related genes p21, p16, CXCL1 (CXC motif chemokine ligand 1), MMP13 (matrix metallopeptidase 13), and ICAM1 (intercellular adhesion molecule 1) was significantly higher in the mice fed the diet of the present invention than in the mice fed chow before the end of the diet feeding period (20 weeks) (FIGS. 8e to 8i, *p<0.05, **p<0.01, ***p<0.005, Student's t-test).

### Change in Senescence-Related Enzyme Protein in Liver Tissue

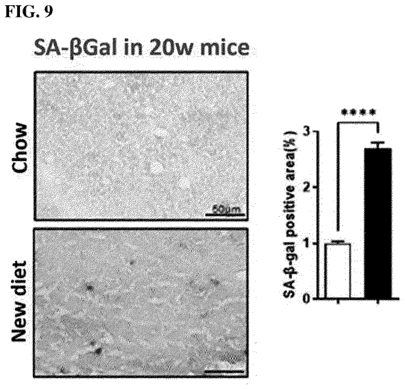

SA-β-gal staining was performed to detect senescence-related β-galactosidase expression using tissue slides fixed with formalin. As a result, it was confirmed that the number of cells stained with SA-β-gal significantly increased in the group fed the diet of the present invention compared to the group fed chow (FIG. 9, Student's t-test). Thereby, it was confirmed that NASH accompanied by senescence could be reproduced in the group fed the diet of the present invention.

### Changes in T Cell and Macrophage Profiling

At the end of the diet feeding, the liver tissue was harvested and minced, and single cells were obtained using the gentle MACS^{™} dissociator (Miltenyi Biotec; 130-0930235, complete medium: high glucose + 10% FBS + 1% penicillin-streptomycin, collagenase I, Miltenyi Biotec protocol). After secondary separation using a strainer, the cell layer was separated using 40% Percoll (GE Healthcare), and then RBCs were removed using RBC lysis buffer (Biolegend, #420301). Thereafter, FACS analysis on T cells and macrophages was performed using the isolated immune cells, and the antibodies used were as follows.

**[Table 3]**

| **Antibody** | **Fluorochrome** | **Clone** | **Manufacturer** | **Cat#** |
|---|---|---|---|---|
| Fc block | . | 2.4G2 | BD Bioscience | 553142 |
| Fixable viability dye | APC-Cy7 | . | eBioscience | 65-0865 |
| CD4 | PerCP-eF710 | GK1.5 | eBioscience | 46-0041 |
| CD8 | Alexa Fluor 700 | 53-6.7 | BioLegend | 100730 |
| CD44 | FITC | IM7 | eBioscience | 11-0441 |
| rdTCR | PE-Cy7 | eBioGL3 | Invitrogen | 25-5711 |
| CD153 | PE | RM153 | eBioscience | 12-1531 |
| IL-17a | PE | eBio17B7 | eBioscience | 12-7177 |
| IFN-r | APC | XMG1.2 | eBioscience | 12-7311 |
| TNF-A | APC | MP6-XT22 | eBioscience | 17-7321 |
| TOX | PE | REA473 | Miltenyi | 130-120-716 |
| F4/80 | PE | T45-2342 | BD Bioscience | 565410 |
| CD11b | PE-Cy7 | M1/70 | BD Bioscience | 552850 |

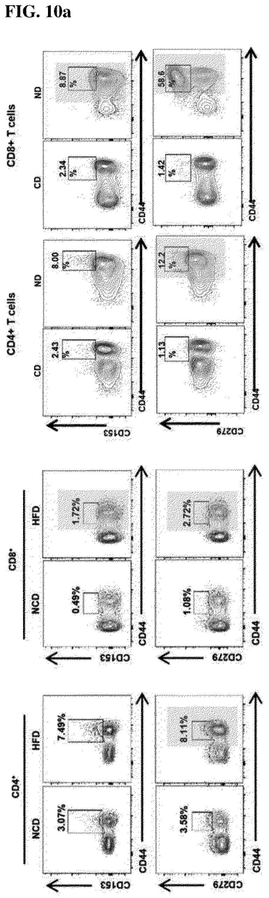

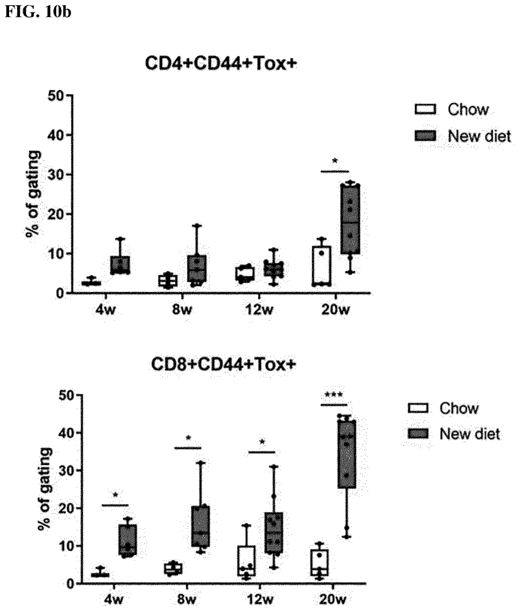

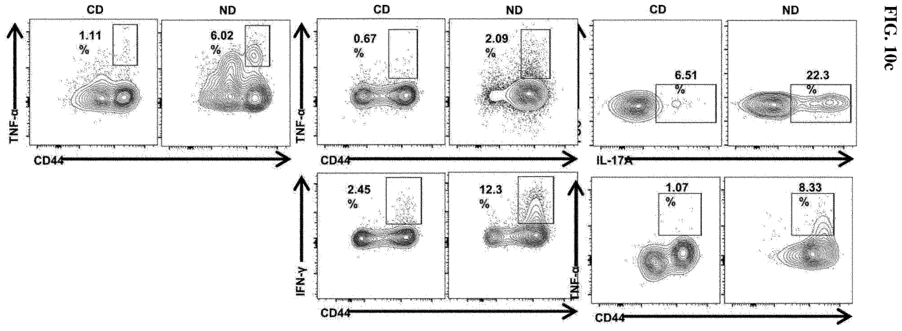

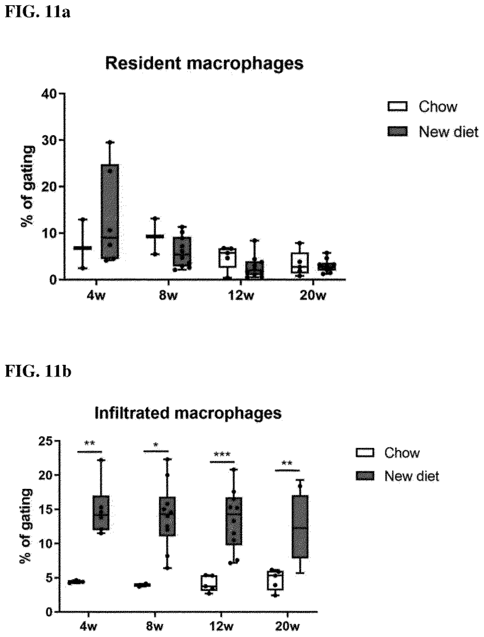

As a result, it was confirmed that the number of senescent T cells significantly increased in the group fed the diet of the present invention compared to the group fed the existing high-fat diet, and this increase was more noticeable in CD8⁺ T cells than in CD4⁺ T cells (FIGS. 10a and 10b). In addition, it was confirmed that the number of T cells expressing inflammation-related genes also increased (FIG. 10c). This is one of the characteristics of human NASH (Nature 592:450-456 (2021)), showing that the dietary composition of the present invention induces NASH in animals more efficiently. Additionally, as a result of macrophage profiling using F4/80 and CD11b antibodies, it was confirmed again that the degree of macrophage infiltration was significantly higher in the group fed the diet of the present invention than in the group fed the existing high-fat diet, suggesting that the dietary composition of the present invention can more effectively reproduce the characteristics of NASH in animals (FIGS. 11a and 11b).

### Changes in Body Weight Over 50 Weeks

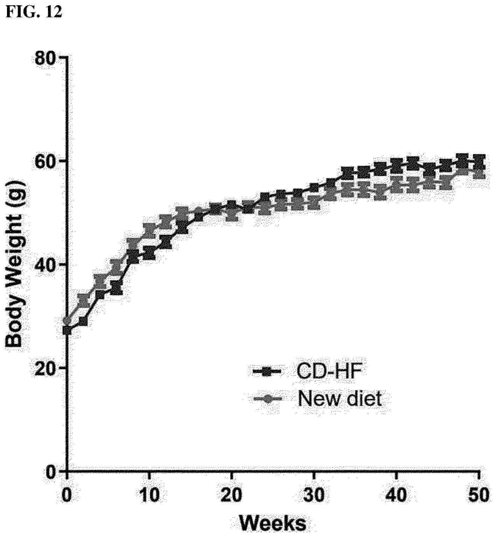

12-week-old C57BL6/J mice were fed CDHFD (choline-deficient high fat diet, N = 14) or the diet of the present invention (N = 13) over 50 weeks, and the body weight of each mouse was measured at the same time every week. As a result, it was confirmed that the body weight gain by the diet of the present invention was higher than that by CDHFD at the beginning of the diet feeding, but decreased rather than increased at week 20 (the time when the diet of the present invention was optimized for the NASH model) (FIG. 12). This was believed to be a pathological phenomenon caused by acceleration of the progression of liver disease (liver cancer) in the group fed the diet of the present invention compared to the group fed CDHFD as the diet feeding period became longer.

### Induction of Intrahepatic Tumor

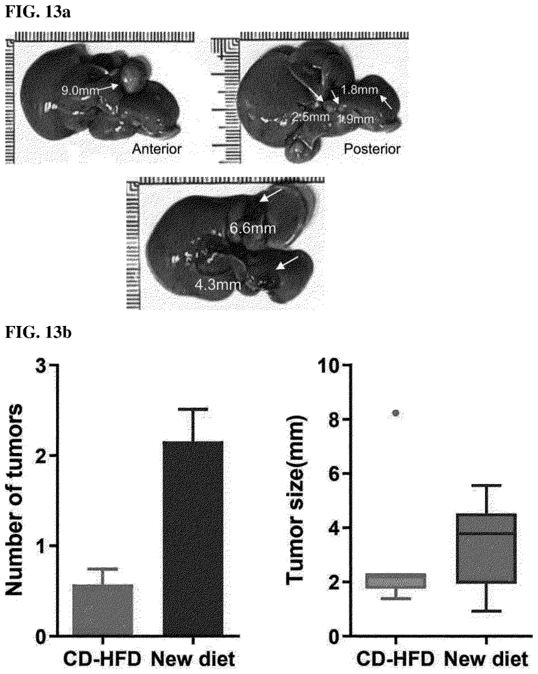

12-week-old C57BL6/J mice were fed the diet of the present invention (N=13) over 50 weeks and then sacrificed to confirm the formation of tumors in the liver tissue (FIG. 13a). Abnormal protrusions (1 to 2 mm or more) appearing on the surface of the liver are a morphological feature of the tumor, and their number was measured visually and the diameter of each protrusion was measured using a ruler. As a result, it was confirmed that the tumor incidence rate (calculated based on the presence or absence of size-measurable protrusions judged to be tumors) was 50% in the group fed CD-HFD for 50 weeks, but 84.62% in the group fed the diet of the present invention for 50 weeks, indicating that the tumor incidence rate significantly increased by about 70% in the diet of the present invention compared to CD-HFD. In the CD-HFD group, 1 out of 14 mice (7%) had a tumor diameter greater than the average (2.7 mm), whereas in the group fed the diet of the present invention, 6 out of 13 mice (46%) had a tumor diameter greater than the average (3.4 mm). Thus, from the tumor size, it could be seen that the diet of the present invention could induce tumor development in the liver more efficiently (FIG. 13b).

Although the present invention has been described in detail with reference to the specific features, it will be apparent to those skilled in the art that this description is only of a preferred embodiment thereof, and does not limit the scope of the present invention. Thus, the substantial scope of the present invention will be defined by the appended claims and equivalents thereto.

### Industrial Applicability

The present invention relates to a composition for inducing liver disease in a mammal and a mammal with a liver disease induced using the same. The present invention may be useful as a means for developing a therapeutic agent for chronic liver disease as well as studying the molecular and circulatory mechanisms of each stage of liver disease.

### Sequence Listing Free Text

SEQ ID NO. 1: GCTACGACGTGGGCTACAG (TNF alpha forward primer)
SEQ ID NO. 2: CCCTCACACTCAGATCATCTTCT (TNF alpha reverse primer)
SEQ ID NO. 3: TTAAAAACCTGGATCGGAACCAA (MCP1 forward primer)
SEQ ID NO. 4: GCATTAGCTTCAGATTTACGGGT (MCP1 reveres primer)
SEQ ID NO. 5: TAAGGGTCCCCAATGGTGAGA (Col1a1 forward primer)
SEQ ID NO. 6: GGGTCCCTCGACTCCTACAT (Col1a1 reverse primer)
SEQ ID NO. 7: GTCCCAGACATCAGGGAGTAA (alpha SMA forward primer)
SEQ ID NO. 8: TCGGATACTTCAGCGTCAGGA (alpha SMA reverse primer)
SEQ ID NO. 9: GCAGACCACAGCGTATCCA (p21 forward primer)
SEQ ID NO. 10: AACAGGTCGGACATCACCAG (p21 reverse primer)
SEQ ID NO. 11: CCCAACGCCCCGAACT (p16 forward primer)
SEQ ID NO. 12: GCAGAAGAGCTGCTGCTACGTGA (p16 reverse primer)
SEQ ID NO. 13: ACCGAAGTCATAGCCACACTC (CXCL1 forward primer)
SEQ ID NO. 14: CTCCGTTACTTGGGGACACC (CXCL1 reverse primer)
SEQ ID NO. 15: AAGGGGATAACAGCCACTACAA (MMP13 forward primer)
SEQ ID NO. 16: ACCAACATAAAAATTAAGCCAAAT (MMP13 reverse primer)
SEQ ID NO. 17: CTTCCAGCTACCATCCCAAA (ICAM1 forward primer)
SEQ ID NO. 18: CTTCAGAGGCAGGAAACAGG (ICAM1 reverse primer)

## Claims

1. A feed composition for inducing in a mammal a liver disease selected from the group consisting of steatohepatitis, hepatic fibrosis, cirrhosis and liver cancer, the composition comprising proteins, carbohydrates, and fats accounting for 13 to 17%, 38 to 42%, and 43 to 47%, respectively, of total calories in the composition.

2. The composition of claim 1, wherein the composition comprises, based on the total weight of the composition, 15 to 19 w/w% of casein and 0.2 to 0.3 w/w% of L-cysteine.

3. The composition of claim 2, wherein the composition further comprises, based on the total weight of the composition, 21 to 25 w/w% of fructose and 10 to 12 w/w% of sucrose.

4. The composition of claim 3, wherein the composition further comprises, based on the total weight of the composition, 16 to 20 w/w% of lard.

5. The composition of claim 4, wherein the composition further comprises, based on the total weight of the composition, 0.5 to 0.7 w/w% of cholesterol.

6. The composition of claim 1, wherein the composition does not comprise choline.

7. The composition of claim 1, wherein the composition increases expression of at least one gene, selected from the group consisting of α-SMA, COL1A1, TNF-α, MCP-1, p21, p16, CXCL1, MMP13, and ICAM1, in liver tissue.

8. The composition of claim 1, wherein the steatohepatitis is nonalcoholic steatohepatitis.

9. The composition of claim 1, wherein the mammal is a rodent animal.

10. A method for producing an animal with an induced liver disease selected from the group consisting of steatohepatitis, hepatic fibrosis, cirrhosis, and liver cancer, the method comprising a step of administering the composition of any one of claims 1 to 9 to a mammal.

11. The method of claim 10, wherein the method is performed by feeding the mammal the composition in an amount of 2.5 to 4 g/kg every day.

12. The method of claim 11, wherein the method is performed by feeding the mammal the composition for 30 to 400 days.

13. A mammal with an induced liver disease selected from the group consisting of steatohepatitis, hepatic fibrosis, cirrhosis, and liver cancer, produced by the method of claim 10.

14. The mammal of claim 13, wherein the mammal exhibits continuous disease progression from steatohepatitis to liver cancer.
